(19) Europäisches Patentamt
European Patent Office
Office européen des brevets

(11) EP 4 787 523 A1

(12) EUROPEAN PATENT APPLICATION

(43) Date of publication:
**05.08.2026 Bulletin 2026/32**

(21) Application number: **25382059.1**

(22) Date of filing: **30.01.2025**

(51) International Patent Classification (IPC):
***H01M 10/0525*** *(2010.01)* ***C07D 493/04*** *(2006.01)*
***H01M 10/0565*** *(2010.01)* ***C08F 122/10*** *(2006.01)*
***C08L 33/06*** *(2006.01)*

(52) Cooperative Patent Classification (CPC):
**H01M 10/0565; C07D 493/04; C08F 122/10; C08L 33/062; H01M 10/0525**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Basque Center for Macromolecular Design and Engineering, POLYMAT Fundazioa**
  **20018 Gipuzkoa (ES)**
- **Universidad del País Vasco/Euskal Herriko Unibertsitatea**
  **48940 Leioa, Vizcaya (ES)**

(72) Inventors:
- **DEL OLMO, Rafael**
  **20018 Donostia - San Sebastián (Gipuzkoa) (ES)**
- **SANTINO, Federica**
  **20018 Donostia - San Sebastián (Gipuzkoa) (ES)**
- **DE MORAIS ZANATA, Daniela**
  **20018 Donostia - San Sebastián (Gipuzkoa) (ES)**
- **VILLALUENGA, Irune**
  **20018 Donostia - San Sebastián (Gipuzkoa) (ES)**

(74) Representative: **ABG Intellectual Property Law, S.L.**
**Avenida de Burgos, 16D**
**Edificio Euromor**
**28036 Madrid (ES)**

# (54) BIO-BASED POLYMER ELECTROLYTES AND METHOD OF PREPARATION THEREOF

(57) The present invention refers to solid and gel polymer electrolytes comprising bio-based monomers and a lithium salt. The invention also refers to a method to obtain the solid and gel polymer electrolytes in presence of a polymerization initiator. Furthermore, plasticizers and co-monomers may be added to tune the electrochemical and rheological properties of the electrolyte, which showed high resistance of lithium dendrites growth and high electrochemical stability at high voltage and against lithium metal in electrochemical cells in long term at room temperature.

EP 4 787 523 A1

## Description

### FIELD OF THE INVENTION

The present invention relates to the field of polymer electrolytes for electrochemical applications, particularly to the preparation of solid and gel polymer electrolytes (SPEs and GPEs) and their application in solid-state lithium batteries.

### BACKGROUND

The main key parameters for battery systems are energy density, cost (€/kWh), cycle life, safety, environmental sustainability and recyclability. The decarbonization and electrification of transport sector has brought the need for longer autonomy vehicles, hence higher energy density batteries than current lithium-ion batteries (LIBs) (250 Wh $kg^{-1}$) are demanded for longer autonomy. Among the alternative technologies, lithium metal batteries (LMBs) are considered the next generation of batteries with an energy density of 450 Wh $kg^{-1}$ (Zhai, P. et al., Adv. Energy Mater. 2020, 10 (34), 1-32). As a consequence of the high reactivity of lithium metal, this will form a solid electrolyte interphase (SEI) by the reaction with any available electrolyte upon contacting. The use of devices based on lithium metal is very influenced from the electrolyte optimization (Zhang, C. et al., J. Mater. Chem. A 2021, 9 (23), 13388-13401). The SEI will evolve through the electrochemical reduction of electrolyte components during the charge of battery (Li, S. et al., Adv. Mater. 2018, 30 (17), 1-29.). The formation of a stable SEI accompanied with the homogeneity of lithium-ion plating deposition will determine final performance of the battery (Adenusi, H. et al., Adv. Energy Mater. 2023, 13 (10)). The growth of lithium dendrites through the heterogeneous deposition can lead either to 1) short-circuit and safety hazards or 2) severe side reactions with the electrolyte that form dead lithium and electrolyte consumption causing an impeded ion transport and hence, capacity fading (Aslam, M. K. et al., Nano Energy 2021, 86 (March), 106142). Consequently, huge efforts have been made to optimize the SEI formation for high-performance long-term cycling of LMBs.

A large number of additives and plasticizers have been evaluated in the field of liquid electrolytes looking for combinations that enable a stable SEI formation (Li, S. et al., Energy Storage Mater. 2020, 32, 306-319). However, the use of highly-concentrated electrolytes or typical carbonates, ethers and fluorinated compounds as main solvents has not solved yet the lithium metal instability (Yang, H. et al., Energy Storage Mater. 2020, 30,, 113-129). Consequently, new chemistries have been studied for both solvents and solutes pursuing long cycle life devices. Recently, isosorbide dimethyl ether (IDE) has been probed as non-flammable electrolyte for silicon technology with a very stable cycling. IDE is known to be nontoxic and environmentally benign as well. A recent work studied the use of 1 M LiFSI in IDE combined with 1,1,1-trifluoroethyl-1,1,2,2-tetrafluoropropyl ether (TTE) to tune the viscosity exhibited a 90 % of capacity retention for 100 cycles for silicon anodes (Johnson, N. M. et al., ACS Energy Lett. 2022, 7 (2), 897-905). The work highlighted that IDE featured high stability on its own, however its combination with a low-viscosity co-solvent (such as TTE) in a liquid electrolyte can improve discharge capacities without altering the high stability of the IDE. As a result, considering its poor exploration, IDE constitutes a promising and sustainable plasticizer with interesting attributes such as its cost, commercial availability and non-toxicity.

On the other hand, solid electrolytes may act as physical barrier for the lithium dendrites growth (Kang, S. et al., Energy Mater. 2023, 3 (5)). In this regard, gel polymer electrolytes (GPEs) emerged as a solution with a good compromise between mechanical properties and sufficient ion diffusion. Several strategies can be employed to develop GPEs from homopolymers and copolymers towards crosslinked or block copolymer or random copolymer membranes, which are considered promising candidates due to their scalability, and chemical/mechanical stability. Solid polymer electrolytes (SPEs) and gel polymer electrolytes (GPEs) may therefore represent a promising alternative to flammable liquid electrolytes which pose serious safety concerns in current batteries. Thus, the preparation of efficient solid state polymer electrolytes with optimal rheological and electronic properties is still a challenge in the field.

### BRIEF DESCRIPTION OF THE INVENTION

The inventors have discovered new bio-based polymer materials comprising a (co)polymer as organic component and at least a lithium salt. The innovative and eco-friendly GPEs and SPEs described herein are promising electrolyte candidates for lithium metal batteries. These electrolytes may further comprise a plasticizer. Focusing on the enhancement of ion transport and cell performance, the lithium salt and optional plasticizer concentration could span across a wide range in high performance devices. Besides, the mechanical properties which are key parameters for the resistance of lithium dendrites growth were also found to be improved. The new electrolytes present high ionic conductivity (>$10^{-5}$ S $cm^{-1}$ at 30 °C), high storage modulus, no sign of short circuit up to 0.4 mA $cm^{-2}$ and very stable plating-striping shape for 2000 h at 0.1 mA $cm^{-2}$. The new electrolyte materials were tested in sustainable full cells with cobalt-free LMFP active material at room temperature and showed electrochemical stability at high voltage and against lithium metal as a proof of concept. The proposed GPEs exhibited a very stable cycling in cobalt-free LMFP full cells during 50 cycles at C/20. Thus, the

invention overcomes most of the problems related to the use of flammable and unstable liquid electrolytes and provides more sustainable and high-performance energy storage solutions as solid polymer electrolytes for lithium metal batteries.

In a first aspect, the invention refers to a mixture comprising:

i) a monomer of formula:

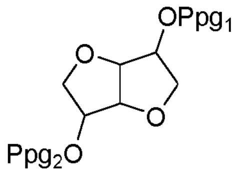

wherein $Ppg_1$ and $Ppg_2$ are independently a polymerizable group, preferably $Ppg_1$ and $Ppg_2$ are independently selected from an olefin-containing or an isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene or isocyanate; and

ii) a lithium salt.

In a second aspect, the invention refers to the use of the mixture of the first aspect in the preparation of a solid or gel polymer electrolyte.

In a third aspect, the invention refers to a method for preparing a solid or gel polymer electrolyte comprising polymerizing in the presence of a polymerization initiator, preferably by irradiating and/or heating, a monomer of formula:

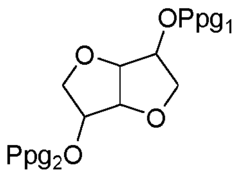

wherein $Ppg_1$ and $Ppg_2$ are independently a polymerizable group, preferably $Ppg_1$ and $Ppg_2$ are independently selected from an olefin-containing or an isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene or isocyanate, wherein a lithium salt is added before, during or after polymerizing of the monomer; particularly, the third aspect refers to a method comprising the steps of:

i) mixing a monomer as defined in the first aspect of the invention with a lithium salt and a polymerization initiator; and

ii) irradiating and/or heating the mixture from step i), causing thereby the polymerization of the monomer.

In a fourth aspect, the invention refers to a solid or gel polymer electrolyte obtainable by method of the third aspect of the invention. Alternatively, the solid or gel polymer electrolyte comprises:

i) a polymer comprising a monomeric unit of formula:

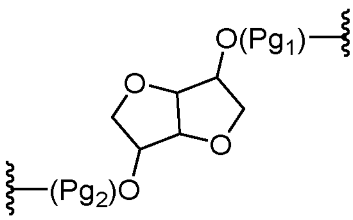

wherein ($Pg_1$) and ($Pg_2$) are groups independently obtained from the polymerization of a polymerizable group, preferably from the polymerization of an olefin-containing or an isocyanate-containing polymerizable group, more preferably from the polymerization of a group selected from acrylate or a C1-C6 alkyl substituted derivative thereof, styrene, diene, maleimide, norbornene, alkylene or isocyanate; and

ii) a lithium salt.

In a fifth aspect, the invention refers to an electrochemical cell or a battery ("electrochemical cell or a battery of the invention") comprising the solid or gel polymer electrolyte of the fourth aspect of the invention; particularly, the electrochemical cell or battery comprises an anode, the solid or gel polymer electrolyte according to the fourth aspect, a cathode and, optionally, a separator.

In a sixth aspect, the invention refers to the use of the electrochemical cell or battery of the invention for storing energy, and more particularly for storing energy in a vehicle, an electronic device or an electrical grid.

A seventh aspect of the invention refers to a combination comprising an electrode for an electrochemical cell or battery, and the mixture of the first aspect of the invention; particularly, the combination comprises an anode and/or a cathode for an electrochemical cell or battery, and the mixture according to the first aspect of the invention.

## DESCRIPTION OF THE FIGURES

**Figure 1.** (a) Ionic conductivity as function of temperature of the prepared electrolytes: 1M LiFSI in IDE and 1M LiFSI in IDE: isosorbide dimethacrylate at 90: 10 wt.%, 80:20 wt.% and 70:30 wt.%. (b) Ionic conductivity as function of temperature of the prepared electrolytes: 1M LiFSI in IDE and with IDE: isosorbide dimethacrylate = 80:20 wt.% at different LiFSI content (1M, 1.25M and 1.5 M in IDE).

**Figure 2.** Second heating DSC curves of 125CISB.

**Figure 3.** SEM cross-sectional image of 125CISB.

**Figure 4.** a) Frequency sweeps (0.1 % strain) and b) oscillatory strain sweep (1 Hz) of CISB electrolytes strain sweep. All the experiments were carried out at 30 °C.

**Figure 5.** Electrochemical stability of 1M LiFSI in IDE (dotted line, 4.6V) and 125CISB (solid line, 4.5V) at 25 °C measured in Li||SS cells at 1 mV $s^{-1}$.

**Figure 6.** Galvanostatic cycling (0.1 mA $cm^{-2}$) of CISB electrolytes in lithium symmetric cells. Inset depicts a zoom-in around 1500 h.

**Figure 7.** Electrochemical performance of 125CISB in full cells. a) Specific capacity and b) voltage profile in Li||LMFP cells at C/20 (0.044 mA $cm^{-2}$).

## DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

Throughout the description and claims the word "comprises" and variations of the word, are not intended to exclude other technical features, additives, components or steps. Furthermore, the word "comprise" encompasses the cases of "consist of' and "consists essentially of", which independently represent particular embodiments. Additional objects, advantages and features of the invention will become apparent to those skilled in the art upon examination of the description or may be learned by practice of the invention. For the purposes of the invention, any ranges given include both the lower and the upper end-points of the range. Ranges or values given, such as temperatures, times, molar ratio, volume ratio and the like, should be considered approximate when they are defined by the term "about" (i.e. with a 5% margin of variation around indicated point).

The first aspect of the invention refers to a mixture comprising:

i) a monomer of formula:

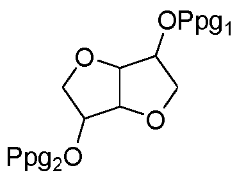

wherein $Ppg_1$ and $Ppg_2$ are independently a polymerizable group, preferably $Ppg_1$ and $Ppg_2$ are independently selected from an olefin-containing or isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene or isocyanate; and

ii) a lithium salt.

The mixture of the first aspect is a polymerizable mixture, meaning that, the monomer therein is prone to polymerize under certain conditions (e.g. by heating and/or by irradiating light). The polymerization is achievable by virtue of the presence of the polymerizable group in the monomer. The term "monomer" (in the context of polymers) refer to the structural motif before being subjected to polymerisation. It is an independent molecular entity which can be polymerised into a polymer. On the other hand, it is commonplace in the art to refer to "monomeric units" as structural motifs in a polymer that stems from a monomer that has been subjected to polymerisation according to the structure of the monomer, even though the monomeric unit itself may no longer show exactly the same structure as the monomer. Thus, for instance, "styrene monomeric unit" actually refers to a monomeric unit derived from a styrene monomer by polymerisation, even though the styrene monomeric unit no longer comprises the alkene group of styrene. The same applies to acrylate or methacrylate monomeric units as well as the other monomeric units mentioned above.

The abbreviation "Ppg" refers to the polymerizable groups in the monomer before polymerization. The abbreviation "Pg" refers to the "Ppg" group in the corresponding monomeric unit after polymerization. The skilled person is well aware of which monomers correspond to which monomeric units. Similarly, the skilled person is well aware of how to convert monomers into corresponding monomeric units by a process of polymerization. It is understood that the monomers must be polymerizable, i.e. they must comprise a functional group which can react with other monomers in a polymerization reaction. The polymerization may require stimuli (heat, light, etc.) and/or a catalyst/initiator largely depending on the specific monomer.

The "acrylate or a C1-C6 alkyl substituted derivative thereof' refers to acrylate and derivatives thereof where a C1-C6 alkyl, preferably a C1-C3 alkyl, substitutes one of the hydrogen atoms at the acrylate C=C double bond; preferably the C1-C6 alkyl substitutes the hydrogen atom at the internal carbon of the acrylate C=C double bond; more preferably the C1-C6 alkyl substituted derivative of acrylate is methacrylate.

In the context of the invention, a C1-C6 alkyl means a straight or branched saturated hydrocarbon chain having 1, 2, 3, 4, 5, or 6 carbon atoms, such as methyl, ethyl, n-propyl isopropyl, n-butyl, i-butyl, t-butyl, etc. Preferably, the C1-C6-alkyl is a C1-C3 alkyl (methyl, ethyl, n-propyl isopropyl), more preferably the C1-C6-alkyl is methyl.

In embodiment, the polymerizable groups $Ppg_1$ and $Ppg_2$ include, but are not limited to the following:

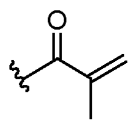
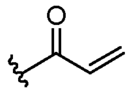
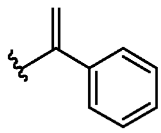
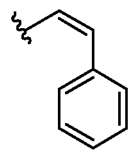
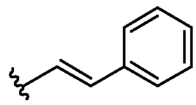

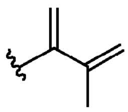
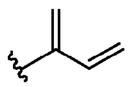
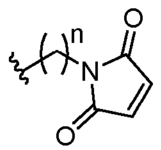
n = 0, 1, 2, 3
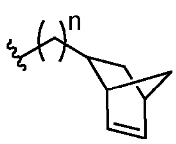
n = 0, 1, 2, 3
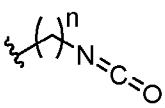
n = 0, 1, 2, 3

In an embodiment, the polymerizable groups $Ppg_1$ and $Ppg_2$ are independently selected from acrylate or a C1-C6 alkyl substituted derivative thereof, styrene, maleimide, norbornene, or isocyanate.

In an embodiment, $Ppg_1$ and $Ppg_2$ are different. In another embodiment, $Ppg_1$ and $Ppg_2$ are the same. Preferably, they are the same.

In a preferred embodiment, $Ppg_1$ or $Ppg_2$, preferably $Ppg_1$ and $Ppg_2$, are independently a C1-C6 alkyl-substituted acrylate derivative, preferably a C1-C3 alkyl-substituted acrylate derivative, more preferably acrylate or methacrylate.

In a most preferred embodiment, $Ppg_1$ and $Ppg_2$ are methacrylate.

In a preferred embodiment, the monomer bicyclic core (i.e. the moiety of the monomer to which the Ppg1 and Ppg2 groups are bound) is selected from isosorbide, isomannide, or isoidide core.

In a preferred embodiment, the monomer is selected from isosorbide dimethacrylate, isomannide dimethacrylate, or isoidide dimethacrylate.

In a preferred embodiment, the monomer is isosorbide dimethacrylate (IDE).

In an embodiment, the mixture comprises a lithium salt. In an embodiment, the lithium salt is an organic lithium salt. The organic lithium salt may be selected from $LiN(SO_2CF_3)_2$ (LiTFSI), $LiN(SO_2F)_2$ (LiFSI), $LiN(SO_2CF_3)(SO_2F)$, $LiN(C_2F_5SO_2)(SO_2F)$, $LiB(C_2O_4)_2$, $LiBF_4$, $LiBF_2(C_2O_4)$, $LiC(SO_2CF_3)_3$, $LiPF_3(C_2F_5)_3$, $LiCF_3SO_3$, n-BuLi, MeLi, t-BuLi,

LiOMe or a mixture thereof; preferably, the organic lithium salt is LiFSI.

In an embodiment, the lithium salt comprised in the mixture of the invention is a single-ion conductor salt. The term single-ion conductor salt refers to a salt in which the anion of the salt will be confined within the gel or solid polymer electrolyte following polymerization of the mixture as described in the third aspect of the invention. Single-ion conductor salts are well known in the art, such as from Honda, Functionality of Molecular Systems Volume 2 From Molecular Systems to Molecular Devices, Springer-Verlag Tokyo 1999, Chapter 2.1.2; Hatada et al., Macromolecular design of polymeric materials, Marcel Dekker, Inc., 1997, Chapter 33. The confinement of the salt anion within the gel or solid electrolyte or within a compartment thereof prevents or reduces migration of the anion to or towards other compartments of the electrochemical cell offering greater solubility for the anion, leading to the loss of salt from the gel or solid polymer electrolyte or compartment thereof, hence to a salt concentration gradient within the electrochemical cell, and ultimately to poor electrochemical performance.

The confinement of the lithium salt anion can be achieved through different means, such as: i) by covalent bonding of the anion to the monomer and/or to the co-monomer comprised in the mixture of the invention, either directly or through a linker moiety; ii) by including the anion in a further polymer comprised in the gel or solid polymer electrolyte; iii) by grafting of the anion on the surface of an inorganic filler further comprised in the gel or solid electrolyte.

In a preferred embodiment, when the lithium salt comprised in the mixture of the invention is a single-ion conductor salt, the anion of the salt is one which is covalently bound to the monomer and/or to the co-monomer comprised in the mixture of the invention, either directly or through a linker moiety; or is one which is covalently bound to the monomer and/or to the co-monomer comprised in the mixture of the invention, either directly or through a linker moiety, following polymerization of the mixture as described in the third aspect of the invention. Examples of lithium salts the anion of which may be covalently bound to the monomer and/or to the co-monomer include lithium salts comprising an anion comprising a polymerizable group, which may be a polymerizable group selected from the above described $Ppg_1$ and Ppg2 groups. Examples of such anions are 4-styrenylsulfonyl(trifluoromethane sulfonyl) imide or 3-sulfonyl (trifluoromethylsulfonylimide) propyl methacrylate salts), which are commercially available from Specific Polymers.

The cation of the single-ion conductor salt is a lithium cation.

In a preferred embodiment of the first aspect, the mixture further comprises a plasticizer. The plasticizer further improves the ionic conductivity, the flexibility, and mechanical properties of the electrolyte material obtained from the mixture of the first aspect. Preferred plasticizers are in a liquid form at ambient pressure and temperature. Examples of plasticizers are organic solvents; these include, but are not limited to, organic carbonates, esters, ethers, ionic liquids, polyethylene glycol (PEG) derivatives, sulfones, or mixtures thereof. Typical organic carbonates used as plasticizers are ethylene carbonate (EC), propylene carbonate (PC), dimethyl carbonate (DMC), diethyl carbonate (DEC), methyl ethyl carbonate (MEC) among others. Typical esters used as plasticizers are diethyl phthalate (DEP), dibutyl phthalate (DBP), dimethyl terephthalate (DMT), triethyl phosphate (TEP) among others. Typical ethers used as plasticizers are diethyl ether; dimethoxyethane (DME); tetraethylene glycol dimethyl ether (TEGDME); 1,4-dioxane; crown ethers; alkyl ethers according to the formula:

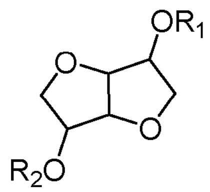

wherein $R_1$ and $R_2$ are independently selected from H, $C_1$-$C_{10}$ alkyl groups, fluorosulfate, sulfonamide, phenyl, provided that $R_1$ and $R_2$ cannot be both H; or mixture thereof; preferably, the alkyl ethers according to the above formula are alkyl ethers of isosorbide, isomannide, isoidide or a mixture thereof.

In particular the alkyl ethers of isosorbide, isomannide, and/or isoidide are mono-alkyl or di-alkyl ethers as one or both -OH groups of the isosorbide, isomannide, and/or isoidide diols can be subjected to etherification, thus giving rise to the following formulas (stereochemistry not shown):

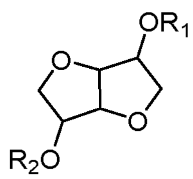

In the above structure, $R_1$ and $R_2$ are independently selected from H and C1-C10 alkyl groups, preferably from H

and C1-C8 alkyl, more preferably from H and C1-C6 alkyl, even more preferably from H, methyl and ethyl, provided that $R_1$ and $R_2$ cannot be both H. In a preferred embodiment, the alkyl ethers of isosorbide, isomannide, and/or isoidide are mono- or di-methyl ethers according to the following formulas (stereochemistry not shown):

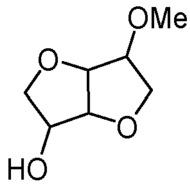 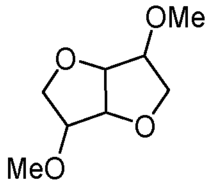 .

Typical ionic liquid-based plasticizers are 1-butyl-3-methylimidazolium tetrafluoroborate (BMIM-$BF_4$), 1-Ethyl-3-methylimidazolium bis(trifluoromethanesulfonyl)imide (EMIM-TFSI), and pyrrolidinium-based ionic liquids. Typical polyethylene glycol (PEG) derivatives used as plasticizers are glymes, polyethylene glycol dimethyl ether (PEGDME), polyethylene glycol diacetate among others. Typical sulfones as plasticizers are methyl sulfone and tetrahydrothiophene 1,1-dioxide (sulfolane).

Finally, some low-volatility solvents can be also used in solid or gel polymer electrolytes, for instance gamma-butyrolactone (GBL), N-methyl-2-pyrrolidone (NMP), and dimethylformamide (DMF).

In a preferred embodiment, the plasticizer is selected from mono- or di-methyl ethers of isosorbide, isomannide, isoidide or mixtures thereof; preferably from mono- or di-methyl ethers of isosorbide, isomannide, isoidide or mixtures thereof; even more preferably, the plasticizer is isosorbide dimethyl ether (IDE).

The mixture can be a physical mixture comprising i) and ii) as defined above or a mixture comprising i) and ii) (as defined above) dissolved or dispersed in a medium, preferably dissolved or dispersed in a solvent medium.

In an embodiment, the physical mixture may be further ground and/or homogenized, such as by mechanical stirring. This solvent-free route offers evident environmental advantages to the route involving a solvent medium.

In an embodiment, the mixture is dissolved in a solvent, in particular an organic solvent. Preferably, the solvent is the plasticizer as it has been defined in any of the embodiments above. The solvent/plasticizer may comprise other miscible solvents (for example other plasticizers).

In an embodiment of the first aspect, the mixture further comprises a co-monomer. Suitable co-monomers are those which may co-polymerize with the $Ppg_1$ and Ppg2 groups of the monomer, such as one according to the formula of the first aspect but with different Ppg1 and/or Ppg2 groups. Other examples of suitable co-monomers are:

- methacrylates (such as methyl methacrylate (MMA), butyl methacrylate (BMA), ethyl methacrylate (EMA), hydroxyethyl methacrylate (HEMA), lauryl methacrylate);
- acrylates (such as ethyl acrylate (EA), butyl acrylate (BA), 2-hydroxyethyl acrylate (HEA), norbornene methyl acrylate);
- vinyl monomers (such as styrene, vinyl acetate (VA), vinyl chloride (VC))
- functional monomers such as glycidyl methacrylate (GMA), which adds epoxy groups for cross-linking or chemical reactivity, and N-Isopropylacrylamide (NIPAAM);
- fluorinated monomers such as 2,2,2-trifluoroethyl methacrylate and perfluoroalkyl methacrylate;
- silane-based monomers such as 3-methacryloxypropyltrimethoxysilane (MPS) and vinyltrimethoxysilane;
- amine-containing monomers such as dimethylaminoethyl methacrylate (DMAEMA) and aminoethyl methacrylate;
- lactones;
- dienes;
- crosslinking monomers such as ethylene glycol dimethacrylate (EGDMA) and divinylbenzene (DVB);
- other specific methacrylate monomers such as polyethylene glycol) methacrylate (PEGMA), cholesteryl methacrylate and isobornyl methacrylate (IBOMA).

In a preferred embodiment, the co-monomer is selected from myrcene, styrene, lactone, methacrylate or acrylate of ethylene oligomers, norbornene methyl acrylate, lithium 3-sulfonyl(trifluoromethane sulfonyl) imide propyl methacrylate, lithium sulfonyl(trifluoromethane sulfonyl)imide styrene.

In an embodiment of the first aspect, the mixture further comprises a polymerization initiator.

In one embodiment, the polymerization initiator is selected from the group consisting of thermal initiators, radical initiators, and photoinitiators, depending on the desired polymerization conditions and target properties of the polymer. Thermal initiators are compounds that decompose at elevated temperatures to produce radicals that initiate polymerization; specific examples include azobisisobutyronitrile (AIBN) and benzoyl peroxide. Radical initiators, which generate free radicals under various conditions to drive polymerization, include compounds such as dicumyl peroxide and t-butyl peroxybenzoate. Photoinitiators are light-sensitive compounds that generate radicals or cations upon exposure to

ultraviolet (UV) or visible light, enabling photopolymerization processes; notable examples are 2-hydroxy-2-methylpropiophenone, 1-hydroxycyclohexyl phenyl ketone (also known as Irgacure 2959) and diphenyl(2,4,6-trimethylbenzoyl) phosphine oxide (also known as Lucirin TPO). The choice of initiator is made based on the polymerization method, the nature of the monomer(s), and the specific application requirements, ensuring optimal control over the polymerization process and the properties of the resulting polymer.

The monomer can be found in the mixture in any wt.% with respect to the total weight of the mixture. In an embodiment, the wt.% of monomer is at least 1 wt.%, at least 2 wt.%, at least 3 wt.%, at least 4 wt.%, at least 5 wt.%, at least 6 wt.%, at least 7 wt.%, at least 8 wt.%, at least 9 wt.%, at least 10 wt.% with respect to the total weight of the mixture. In another embodiment, the monomer is comprised in the mixture in an amount comprised between 1 and 50 wt%, preferably between 2 and 45 wt.%, more preferably between 5 and 40 wt.%, even more preferably between 5 and 30 wt.% (lower and upper limits included) with respect to the total weight of the mixture.

The lithium salt is preferably comprised in the mixture at a concentration comprised between 0.1 M and 5M, more preferably between 0.5 and 2.5 M, even more preferably between 1.0 and 1.5 M. Alternatively, the lithium salt can be found in the mixture in an amount comprised between 5 and 40 wt.%, preferably between 7.5 and 35 wt.%, even more preferably between 10 and 30 wt.% with respect to the total weight of the mixture. The same above embodiments related to the lithium salt apply to the concentration or wt.% of the single-ion conductor salt.

The optional plasticizer may be found in the mixture in any wt.% with respect to the total weight of the mixture. In an embodiment, the wt.% of the plasticizer is at least 10 wt.%, at least 20 wt.%, at least 30 wt.%, at least 40 wt.%, at least 50 wt.% with respect to the total weight of the mixture. In another embodiment, the plasticizer is comprised in the mixture in an amount comprised between 10 and 95 wt%, preferably between 20 and 90 wt.%, more preferably between 30 and 90 wt.%, even more preferably between 50 and 80 wt.% (lower and upper limits included) with respect to the total weight of the mixture. Most preferably, the plasticizer is comprised in the mixture in an amount of about 80 wt.% with respect to the total weight of the mixture.

The optional co-monomer may be found in the mixture in any wt.% with respect to the total weight of the mixture. In an embodiment, the wt.% of the optional co-monomer is at least 1 wt.%, at least 2 wt.%, at least 3 wt.%, at least 4 wt.%, at least 5 wt.%, at least 6 wt.%, at least 7 wt.%, at least 8 wt.%, at least 9 wt.%, at least 10 wt.% with respect to the total weight of the mixture. In another embodiment, the optional co-monomer is comprised in the mixture in an amount comprised between 1 and 50 wt.%, preferably between 2 and 45 wt.%, more preferably between 5 and 40 wt.%, even more preferably between 5 and 30 wt.% (lower and upper limits included) with respect to the total weight of the mixture.

The molar ratio between monomer and optional co-monomer may vary between 90:10 to 10:90, preferably between 75:25 to 25:75, more preferably the molar ratio is of about 50:50.

The optional polymerization initiator may be found in the mixture in an amount comprised between 0.1 and 10 wt.%, preferably between 0.5 and 7.5 wt.%, even more preferably between 1.0 and 5 wt.%, even more preferably at about 3.0 wt.% with respect to the weight of the monomer.

In a particular embodiment, the mixture of the first aspect comprises:

i) a monomer of formula:

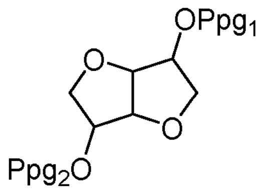

wherein $Ppg_1$ and $Ppg_2$ are independently a polymerizable group, preferably $Ppg_1$ and $Ppg_2$ are independently selected from an olefin-containing or an isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene or isocyanate;

ii) a lithium salt;

iii) optionally, a co-monomer;

and

iv) a plasticizer;

wherein the sum amount of monomer and co-monomer, if present, is comprised between 5 and 30 wt.% (lower and upper limits included) with respect to the total weight of the mixture;

wherein the amount of lithium salt is comprised between 5 and 40 wt.% with respect to the total weight of the mixture; and

wherein the amount of plasticizer is comprised in an amount comprised between 50 and 80 wt.% (lower and upper limits included) with respect to the total weight of the mixture.

In a particular embodiment, the mixture comprises, in particular consists of:

i) a monomer of formula:

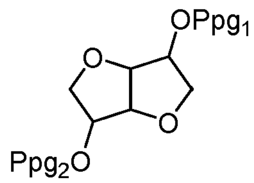

wherein $Ppg_1$ and $Ppg_2$ are independently a polymerizable group, preferably $Ppg_1$ and $Ppg_2$ are independently selected from an olefin-containing or an isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene or isocyanate;
ii) a lithium salt;
iii) optionally, a co-monomer;
iv) a plasticizer; and
v) a polymerization initiator.

In a more particular embodiment, the mixture consists of:

i) a monomer selected from isosorbide dimethacrylate, isomannide dimethacrylate, isoidide dimethacrylate;
ii) a lithium salt;
iii) optionally, a co-monomer;
iv) a plasticizer; and
v) a polymerization initiator.

In a second aspect, the invention refers to the use of the mixture of the first aspect in the preparation of a solid or gel polymer electrolyte.

In a third aspect, the invention refers to a method for preparing a solid or gel polymer electrolyte comprising polymerizing in the presence of a polymerization initiator, preferably irradiating and/or heating, a monomer of formula:

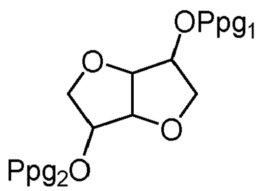

wherein $Ppg_1$ and $Ppg_2$ are independently a polymerizable group, preferably $Ppg_1$ and $Ppg_2$ are independently selected from an olefin-containing or an isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene or isocyanate, wherein a lithium salt is added before, during or after polymerization of the monomer. In an embodiment, the lithium salt is added before polymerization. In an embodiment, the lithium salt is added after polymerization.

In an embodiment, the method of the third aspect comprises the steps of:

i) mixing a monomer of formula:

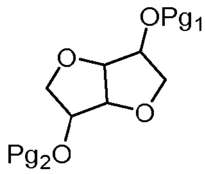

wherein $PG_1$ and $PG_2$ are independently a polymerizable group, preferably $Ppg_1$ and $Ppg_2$ are independently selected from an olefin-containing or an isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene, and isocyanate, with a lithium salt and a polymerization initiator; and
ii) irradiating and/or heating the mixture from step i), causing thereby the polymerization of the monomer.

In an embodiment, the method of the third aspect comprises the steps of:

i) irradiating and/or heating in the presence of a polymerization initiator a monomer of formula:

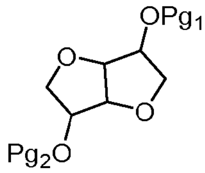

wherein $PG_1$ and $PG_2$ are independently a polymerizable group, preferably $Ppg_1$ and $Ppg_2$ are independently selected from acrylate or a C1-C6 alkyl substituted derivative thereof, styrene, diene, maleimide, norbornene, alkylene, and isocyanate, causing thereby the polymerization of the monomer; and
ii) adding a lithium salt to the polymerised monomer from step i).

In a preferred embodiment of the third aspect, the monomer according to the above formula, the polymerization initiator and the lithium salt are first mixed, neat or dissolved or suspended in a solvent, then the polymerization is carried out by irradiating and/or heating the mixture.

In a preferred embodiment, a plasticizer is added to the mixture of step i). Examples of plasticizers have been described for the first aspect. Polymerization initiators have already been described for the first aspect of the invention. Optional co-monomers as those described for the first aspect may be also used. Particular embodiments relative to amounts of each component of the mixture of step i) have also been described above.

The mixture of step i) may be optionally stirred to ensure homogeneity of the components in the mixture. The stirring may be mechanical o magnetic according to procedures known in the art. The solution may be left under stirring for an appropriate amount of time, typically for at least 1 minutes, at least 2 minutes, at least 5 min; preferably, the solution is stirred between 1 minute and 10 hours, more preferably between 5 min and 1 hour, even more preferably for about 5 min.

Preferably, the mixture of step i) is prepared in the presence of air or under an inert atmosphere so as to avoid degradation of air-sensitive components or side-reactions during polymerization; preferably step i) is carried out under inert atmosphere. Inert atmosphere refers to an atmosphere where oxygen is excluded and comprises gases such as helium, nitrogen, argon and the like.

The method of the third aspect involves a polymerization reaction. Polymerization reactions are well known to the person skilled in the art and may include thermal- or photo-polymerization with or without employing a radical initiator. When polymerization is thermal polymerization, then the heating of the organic-inorganic mixture can advantageously further serve the purpose of achieving polymerization. However, when polymerization is not thermal polymerization, such as when it is photopolymerization, the method of the invention requires subjecting the mixture from step i) to conditions which trigger polymerization, such as exposure to a certain kind of light, the nature of which will depend on the specific monomers or initiator employed.

In an embodiment, the polymerization is a thermal polymerization. The temperature will depend on the monomer and the nature of the mixture of step i). In an embodiment, the temperature is comprised between 10 °C and 100 °C, more preferably between room temperature and 80 °C, even more preferably between room temperature and 60 °C.

In another embodiment, the polymerization of step ii) is a photopolymerization.

The monomer of the invention comprises two polymerizable groups. Hence, unless controlled to avoid it, the polymerization reaction will yield crosslinked polymers.

However, polymerization techniques such as Reversible addition fragmentation chain-transfer polymerization [RAFT] or Nitroxide-mediated radical polymerization [NMP] allow controlling the polymerization, and can particularly

prevent cross-linking. A skilled person would know how these polymerization techniques are carried out. Therefore, the polymer of the invention may be crosslinked or non-crosslinked.

A co-monomer may be comprised in the mixture of the first aspect. When a co-monomer is used, in an embodiment of the third aspect, a co-monomer is polymerized separately and blended with the mixture of the first aspect, before or after polymerization of the monomer. Alternatively, the monomer may be first polymerized (for example by RAFT or NMP), then the co-monomer is added and (co)polymerized. In another embodiment, the monomer and co-monomer are mixed and then co-polymerized. Different polymer structures, such as polymer blends, crosslinked polymers, block copolymers or random copolymers can be obtained through these different polymerization methods.

Preferably, the monomer and the co-monomer are mixed and subsequently co-polymerized.

In an embodiment, the time for polymerization is comprised between 1 second and 24 hours, preferably between 5 seconds and 12 hours.

The polymerization may be carried out on an adequate support (such as a mold) from which it is later removed and used in an electrochemical cell or battery; or directly on an electrode. In a particular embodiment, the mixture comprising a monomer, polymerization initiator and lithium salt or the mixture of monomer and polymerization initiator is applied to an electrode, preferably a cathode, more preferably a cobalt-free cathode. The application of the mixture may be carried out by drop-casting, brush coat, spray coat, doctor blade, dipping or spincoat.

After polymerization of the mixture, and addition of the lithium salt (if not present in the mixture before polymerization), the solid or gel polymer electrolyte of the invention is obtained. The solid or gel polymer electrolyte is a film which may have different thickness depending on the type of polymerization. In an embodiment, the solid or gel polymer electrolyte has a thickness comprised between 1 and 1000 μm, preferably between 10 and 500 μm, more preferably between 200 and 300 μm, even more preferably of about 250 μm.

The solid or gel polymer electrolyte can be further processed before use to the desired shape and thickness. In an embodiment, the method of the third aspect comprises, after polymerization, subjecting the heated mixture to a processing step selected from hot-pressing, extrusion, calendering, lamination, or a combination thereof. This further processing step can be applied when crosslinked polymers are obtained via thermal polymerization.

In a preferred embodiment, the processing step, preferably hot-pressing or extrusion, allows to reach a thickness of the resulting electrolyte comprised between 10 and 100 μm, preferably between 10 and 80 μm, more preferably between 10 and 60 μm.

<u>Polymer electrolyte</u>

In a fourth aspect, the invention refers to a solid or gel polymer electrolyte obtainable by method of the third aspect of the invention.

The solid or gel polymer electrolyte may further comprise a polymerized or cross-linked co-monomer (that is, the co-monomer is polymerized separately or co-polymerized with the monomer) and/or a plasticizer and/or a polymerization initiator as already defined above.

The solid electrolyte obtainable by the method of the invention is characterized by high values of storage (G') and loss (G') moduli. These values can be calculated by employing an ARES-G2 Rheometer.

In an embodiment, the solid electrolyte obtainable by the method of the invention is characterized by a storage modulus (G') comprised between 1 and 1000 MPa, preferably between 10 and 1000 MPa, more preferably between 100 and 1000 MPa, even more preferably of about 1 M Pa.

The hybrid solid electrolyte obtainable by the method of the invention is also characterized by high values of yield stress (σy). This parameter can be calculated by employing an ARES-G2 Rheometer. In an embodiment, the solid electrolyte obtainable by the method of the invention is characterized by a σy comprised between 0.1 and 100 MPa, preferably between 0.5 and 100 MPa, more preferably between 1 and 10 MPa. The higher yield stress values are advantageous in terms of cell failure prevention by suppressing the lithium dendrites formation.

Alternatively, the solid or gel polymer electrolyte comprises:

i) a polymer comprising monomeric units of formula:

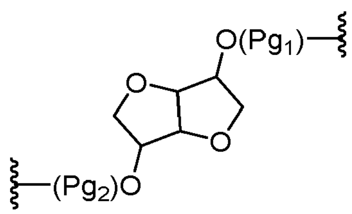

wherein ($Pg_1$) and ($Pg_2$) are groups independently obtained from the polymerization of a polymerizable group, preferably from the polymerization of an olefin-containing or an isocyanate-containing polymerizable groups, more preferably from the polymerization of groups selected from acrylate or a C1-C6 alkyl substituted derivative thereof, styrene, diene, maleimide, norbornene, alkylene, isocyanate; and

ii) a lithium salt.

The embodiments described for the solid or gel polymer electrolyte obtainable by the method of the invention apply also to this alternative aspect.

Electrochemical cell and battery

In a fifth aspect, the invention refers to an electrochemical cell or a battery ("electrochemical cell or a battery of the invention") comprising the solid or gel polymer electrolyte of the fourth aspect of the invention.

In a preferred embodiment, the electrochemical cell or battery is a secondary electrochemical cell or secondary battery. In an embodiment, the electrochemical cell or secondary battery comprises the solid or gel polymer electrolyte as defined in the fourth aspect of the invention, which can be used as anolyte, catholyte and/or separator, preferably as a catholyte.

In an embodiment, the electrochemical cell or battery comprises the solid or gel electrolyte of the invention and a negative electrode (or anode) which is a metal anode, preferably an alkali metal anode, and more preferably a lithium metal anode. In another embodiment, the anode comprises a metal or metalloid suitable for reversibly forming an alloy with metal cations, preferably alkali metal cations, and more preferably lithium cations. The skilled person knows how to select appropriate metals or metalloids suitable for forming the alloy. For instance, examples of metals or metalloids suitable for reversibly forming an alloy with lithium cations are Mg, Al, Zn, Bi, Cd, Sb, Ag, Si, Pb, Sn, or In which in particular can form alloys such as LiMg, LiAl, LiZn, $Li_3Bi$, $Li_3Cd$, $Li_3Sb$, $Li_4Ag$, $Li_{4.4}Si$, $Li_{4.4}Pb$ or $Li_{4.4}Sn$. In an embodiment, the anode comprises such alloys.

In an embodiment, the electrochemical cell or battery comprises the solid or gel electrolyte of the invention and a cathode suitable for reversibly incorporating metal cations, preferably alkali metal cations, and more preferably lithium cations. More specifically, the cathode comprises an active material, which is the component of the cathode enabling said reversible incorporation to take place.

In an embodiment, the cathode comprises metal cations, preferably alkali metal cations, and more preferably lithium cations. In a particular embodiment, the cathode is a cathode obtainable from the intercalation of metal cations in its structure, in particular at its active sites.

In an embodiment, the cathode comprises an active material selected from one of the following:

- a lithium nickel-rich layered oxide of formula $Li_yNi_{1-x}M_xO_2$, wherein M represents at least one metal and $0 \leq x \leq 1$, $0.8 \leq y \leq 1.2$;
- a spinel oxide of formula $LiNi_{2-x}M_xO_4$, wherein M represents at least one transition metal and $0 \leq x \leq 2$;
- a lithium-rich layered oxide of formula $Li_{1+x}M_{1-x}O_2$ wherein M represents at least one transition metal and $0 \leq x \leq 1$;
- a lithium polyanion of formula $Li_2MSiO_4$ wherein M is at least one of Mn, Co or Ni; of formula $LiMPO_4$ wherein M is at least one of Fe, Mn, Co or Ni; of formula $Li_2MP_2O_7$ wherein M is at least one of Mn, Co or Ni; or of formula $Li_3V_2(PO_4)_3$, $Li_2VOP_2O_7$, or $LiVP_2O_7$; and
- a phosphate or sulfate of formula $Li_yMXO_4Z$; wherein y = 0, 1, 2; M = transition metal; X = P, S; Z = F, O, OH.

Cathodes as described herein are commercially available and well known in the art, such as from Li et al., Chem Soc Rev, 2017, 46, 3006-3059, or Lyu et al., Sustainable Materials and Technologies, 2019, 21, e00098. Preferably the cathode is a cobalt-free cathode, more preferably is a lithium manganese iron phosphate (LMFP) cathode.

The cathodes of the present invention may further comprise a conductive carbon material such as carbon black or activated carbon. Preferably, the conductive carbon material is carbon black. The term "carbon black" [C.A.S. NO. 1333-86-4] refers to colloidal aciniform carbon particles produced by the incomplete combustion or thermal decomposition of gaseous or liquid hydrocarbons such as heavy petroleum distillates and residual oils, coal-tar products, natural gas or acetylene. Its physical appearance is that of a black, finely divided pellet or powder.

In an embodiment, the solid or gel polymer electrolyte of the invention acts as a separator in the electrochemical cell or battery. A separator is a medium between the two electrodes of an electrochemical cell that has to fulfill at least two functions. One function is to store and accommodate the electrolyte and, simultaneously, to assure ionic conductivity within the electrodes and between the anode and cathode. The further function of the separator is to electrically insulate the two electrodes from one another, in order to avoid short circuits. The separator is a separating means, usually a plate, positioned between the anode and cathode in the electrochemical cell or battery to avoid the electrical contact between them.

In a particular embodiment, the electrochemical cell of the present invention may comprise the solid or gel polymer electrolyte of the invention and an additional separator.

The separator is in contact with the electrolyte, particularly it is partially or completely contacted with the electrolyte, which favors the flow of ions from one electrode to the other one. Typical separators include, but are not limited to, polymeric membrane mainly based on a polyolefin, such as polypropylene, (PP) polyethylene (PE), or any combination thereof (PP-PE). The polypropylene (or polyethylene or PP-PE) membrane may comprise one or more layer of polypropylene (or polyethylene or PP-PE). In some embodiments, the separator of the electrochemical cell comprises inorganic materials such as a glass fiber.

In a particular embodiment, the electrochemical cell or battery comprises an anode, the solid or gel polymer electrolyte according to the fourth aspect, a cathode and, optionally, a separator.

Uses and applications

In a sixth aspect, the invention refers to the use of the electrochemical cell or battery of the invention for storing energy, and more particularly for storing energy in a vehicle, an electronic device or an electrical grid.

The vehicle can be an automobile, in particular a heavy automobile such as buses or trucks, a rail vehicle, a marine vehicle, an aircraft or a spacecraft.

Preferably, the electronic device is a portable electronic device, such as a laptop, a tablet, a cellular phone, a smart phone or a smart watch.

Preferably, the electrical grid is associated to a solar panel or a wind turbine.

EXAMPLES

The following examples are intended to illustrate but not to limit the disclosed embodiments.

Materials

All the reagents were used without further purification and under glovebox atmosphere. The following materials were employed: lithium bis(fluorosulfonyl)imide (LiFSI) (Solvionic, 99.9 %), isosorbide (Sigma-Aldrich), methacrylic anhydride (Sigma-Aldrich), isosorbide dimethyl ether (IDE, Sigma-Aldrich, >99%), 2-hidroxy-2-methylpropiophenone (Sigma-Aldrich, 97 %), conductive carbon (Super C65, Timcal), poly(vinylidene difluoride) (PVDF Solef® 5130 1000-1100 kg $mol^{-1}$), lithium manganese phosphate (LMFP, Gelon, 99.9 %) .

Example 1. Synthesis of crosslinking monomer

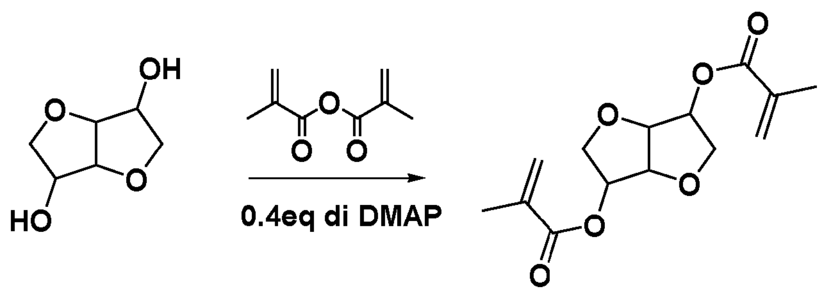


In a two necks flask, isosorbide (1 eq) and 0.4 eq of DMAP were mixed together and the temperature of the mixture was raised to 60 °C under argon conditions. After 2 hours, the mixture became a homogeneous and viscous transparent liquid and the flask was allowed to cool down at room temperature. Then, 2 eq. of methacrylic anhydride were added and the reaction mixture was stirred overnight at 60 °C. The day after the reaction was stopped and the obtained homogeneous liquid was extracted four times with a $NaHCO_3$ saturated aqueous solution. $^1H$ NMR ($CDCl_3$ d = 7.27) d: 6.16 (s, 1H), 6.10 (s, 1H), 5.61 (s, 1H), 5.59 (s, 1H), 5.25 (s, 1H), 5.20 (q, 1H), 4.90 (t, 1H), 4.53 (d, 1H), 3.99 (s, 2H), 3.96 (dd, 1H), 3.89 (d of d, 1H), 1.96 (t, 3H), 1.92 (t, 3H)].

Example 2. Electrolyte preparation

Three liquid electrolytes were prepared. Liquid electrolytes (1.0 M LiFSI in IDE) were stirred overnight before

characterization or addition to the cross-linked system. To develop the crosslinked membranes (CISB), liquid electrolytes were mixed with isosorbide dimethacrylate monomer (as obtained in example 1) at different ratios (90:10, 80:20 and 70:30 wt.% of IDE:isosorbide dimethacrylate, respectively) in an ambar vial with photoinitiator 2-hidroxy-2-methylpropiophenone (3 % w/w of monomer) inside an argon-filled glovebox ($H_2O$ < 0.8 ppm, $O_2$ < 4 ppm). After 5 min stirring, the liquid mixture was drop casted on silicon molds and irradiated with 365 nm lamp for 20 seconds resulting in ~ 250 μm thick solid membranes.

Additional electrolytes were prepared according to the same procedure by fixing the IDE:isosorbide dimethacrylate ratio at 80:20 and further varying the LiFSI concentration at 1.25 M and 1.5 M.

Fresh membranes were prepared before each measurement.

The samples at fixed IDE:isosorbide dimethacrylate = 80/20 wt.% were named throughout the text as 100CISB (1.0M LiFSI), 125CISB (1.25M LiFSI), and 150CISB (1.5M LiFSI). A sample named 50SB was also prepared at 1.0 M LiFSI in IDE and no isosorbide dimethacrylate.

<u>Example 3. Electrolyte characterization</u>

*Ionic conductivity.* Electrochemical impedance spectroscopy (EIS) was performed in Swagelok cells measuring in the temperature range from 30 to 90 °C with an universal oven (Memmert, UF75plus) and using a Biologic potentiostat (SP200) in the range of 1 MHz to 1 Hz with 20 mV of amplitude. The membranes (10 mm in diameter) were assembled glovebox using stainless steel blocking electrodes.

High ionic conductivity was found for 90 wt.% IDE-containing membrane, i.e. $2.5\cdot10^{-4}$ S $cm^{-1}$ at 30 °C (Fig. 1), which is very close to that of liquid 1 M LiFSI in pure IDE (50SB, $5.4\cdot10^{-4}$ S $cm^{-1}$). However, the solid membrane was very soft while 80 wt.% IDE-containing membrane (100CISB, $1.2\cdot10^{-4}$ S $cm^{-1}$ at 30 °C) presented a robust behavior and, consequently, was selected for further optimization.

As observed in Fig. 1b, the ionic conductivity of 100CISB was increased with the addition of more LiFSI up to 1.25 M (125CISB, 1.5 . $10^{-4}$ S $cm^{-1}$ at 30 °C) before dropping with 1.5 M (7.5 . $10^{-5}$ S $cm^{-1}$) probably because of the saturation limit and subsequent salt precipitation.

*Thermal analysis.* Differential scanning calorimetry (DSC) tests were performed to characterize the SPEs as the ion dynamics in polymeric electrolytes are intimately related to the polymer chain motion (Fig. 2). The samples were measured using a PerkinElmer 8500 DSC equipped with an Intracooler III. The samples were cooled down to -90 °C, heated up to 40 °C and held at that temperature for 3 min to erase the thermal history. Subsequently, the samples were cooled down to -90 °C and then heated up again to high temperature. The second heating scan was considered for the study of the thermal properties of the electrolytes.

Accordingly, DSC was employed to determine the glass transition temperature (Tg) of 125CISB. As a result, very low Tg was identified at -74 °C which enables a sufficient segmental motion of the polymeric chain at room temperature in order to facilitate the ion diffusion.

*Scanning electron microscopy (SEM).* SEM analysis analysis was performed in a 3030 Hitachi under vacuum and 15 kV voltage. The membranes were immersed in liquid nitrogen for 1 min in order to obtain a clean cut without membrane deformation. Then, the membranes were stuck to stainless steel substrates with carbon tape and subsequently coated with a gold sputtering. The morphology of the crosslinked membrane as determined by SEM and is shown in Fig. 3. 125CISB presents a dense appearance with no presence of internal bubbles, agglomerations or cracks which could be detrimental for the ion transport through the membrane and therefore, the electrochemical performance. *Rheology.* The toughness of the self-standing membranes is a key parameter for long-term cyclability to avoid dendrite penetration and subsequent short-circuit. Accordingly, rheology experiments were carried out to quantify the mechanical properties of CISB membranes. The rheological properties were determined using a strain-controlled ARES-G2 rotational rheometer (TA Instruments). Samples of 8 mm diameter (250 μm thick) were evaluated in parallel plate geometry at 30 and 90 °C. The experiments were carried out under nitrogen flow and the samples were subjected to strain sweeps to verify their linear viscoelastic region (from 0.004 to 0.2 % at 1 Hz) at room temperature. The frequency sweeps were taken using a shear strain of 0.1 %.

The effect of salt content on the mechanical properties of CISB system was investigated (Fig. 4). It is worth noting they all exhibit G' >> G" in all the range of frequency with a tan $\delta$ around at 0.13 in the three scenarios confirming the observed solid-like behavior. Higher moduli were obtained upon addition of LiFSI in the system: 0.10 MPa (100CISB), 0.11 (125CISB) and 0.15 MPa (150CISB).

The yield stress of the solid electrolytes was also found very relevant in terms of long-term cycling through a different lithium heterogeneous nucleation mechanism varying from dendrites to globular protrusion (Chakraborty, S. et al., ACS Appl. Energy Mater. 2022, 5 (1), 852-861). The results indicated that 125CISB presented the highest value ($3.3\cdot10^3$ Pa) just followed by 150CISB ($2.1\cdot10^3$ Pa) and 100CISB ($1.3\cdot10^3$ Pa). Therefore, 150CISB represents a more resistive candidate for cell failure according to its mechanical properties, requiring higher deformation pressures to ultimately compromise its internal structure.

Example 4. Electrochemical stability and lithium symmetric cells.

Lithium transport number ($t_{Li}$+) was calculated by using Bruce-Vincent method. As observed from Table 1, 1M LiFSI in IDE exhibited a $t_{Li}$+ of 0.30, being comparable to standard liquid electrolytes. Regarding CISB system, 125CISB resulted in 0.27 transport number with a clear increasing trend upon salt addition from 0.14 for 100CISB to 0.30 for 150CISB, as reported for similar systems (Del Olmo, R. et al., J. Power Sources 2024, 621, 235233).

**Table 1.** Results of transport number measurements. The interface resistance is calculated as the sum of $R_{SEI}$ and $R_{ct}$.

| **Δ*E* = 40 mV** | $t_{Li}$+ | $I_0$ (μA) | $I_{SS}$ (μA) | $R_{int,0}$ (kΩ) | $R_{int,SS}$ (kΩ) |
|---|---|---|---|---|---|
| 1M LiFSI in IDE | 0.30 | 6.39 | 1.94 | 1.83 | 5.61 |
| 100CISB | 0.14 | 7.94 | 4.16 | 4.35 | 4.78 |
| 125CISB | 0.27 | 21.80 | 11.92 | 1.31 | 1.39 |
| 150CISB | 0.30 | 5.98 | 2.90 | 3.89 | 4.46 |

The selectivity of the total ionic conductivity for lithium-ion mobility is relevant for a homogeneous lithium plating with significant reduction concentration gradients.

Another important parameter evaluated was the electrochemical window of the studied system for its application in the correct device. Li|||Stainless steel cells were assembled for this purpose to assess the plating-stripping ability, and hence its stability against lithium metal, but also the anodic stability in fresh cells. Lithium circular electrodes were prepared (8 mm in diameter) in an argon-filled glovebox by firstly cleaning lithium metal ribbons with cyclohexane and the use of a nylon brush. CR2032 (Xiamen Tmax) coin cell set up was used to test the SPEs. The assembly was carried out with a manual crimper (Hohsen). Cyclic voltammetry tests were performed after 8 h resting using SP200 Biologic potentiostat from -0.5 to 2.0 V vs $Li^+/Li^0$ at 1 mV $s^{-1}$ at 30 °C. Linear sweep voltammetry was employed to assess the electrochemical stability at 1 mV $s^{-1}$ from open circuit voltage to 6 V vs $Li^+/Li^0$. Liquid electrolytes were evaluated using Celgard 2500 separators while the solid membranes were used as they are. Fig. 5 depicts the electrochemical stability of 1 M LiFSI in IDE and 125CISB. For both systems, the current density increased over cycling demonstrating a good solid electrolyte interface (SEI) formation. In the anodic side, 125CISB showed a slightly lower electrochemical stability (4.5 V vs Li+/Li0) than 1 M LiFSI in IDE (4.6 V vs Li+/Li0), but still sufficiently high to be applied in high-voltage devices such as Li||LMFP.

Li||Li cells were employed to determine the lithium transport number ($t_{Li}$+) of the electrolytes. Given the good compromise between ionic transport and mechanical properties offered by CISB electrolytes, as well as electrochemical stability, they were evaluated galvanostatically in Li||Li symmetric cells in a ramp test (Fig. 5) from 25 to 400 μA $cm^{-2}$. Bruce-Vincent method (Bruce, P. G. et al., Solid State Ionics 1988, 28-30, 918-922) was used after 8 h resting by measuring an initial EIS (10 mV amplitude, 7 MHz - 0.1 Hz) followed by a chronoamperometry (40 mV, 0.1 s resolution) and final EIS (10 mV amplitude, 7 MHz - 0.1 Hz) to calculate $t_{Li}$+ employing the following equation:

$$t_{Li^+} = \frac{I_{SS}(\Delta V - I_0 R_{int,0})}{I_0(\Delta V - I_{SS} R_{int,SS})}$$

where $I_0$ and $I_{SS}$are the current measured at the beginning of the chronoamperometry and in the steady state, respectively and ΔV is the applied potential in the chronoamperometry. A second fresh row of Li||Li cells was employed to perform galvanostatic critical current density in the range of 25-400 μA $cm^{-2}$ (5 min resting between charge and discharge) at room temperature for CISB membranes. Last, a third row of Li||Li cells was destined for long-term cycling at 100 μA $cm^{-2}$ with similar conditions of resting and temperature for CISB electrolytes.

As a result, 125CISB exhibited the lowest overpotential (η) with a proportional increase with the current density applied and no sign of short-circuit in all the range. In contrast, 150CISB and 100CISB showed higher overpotential than 125CISB. Indeed, there was a clear jump in η from 250 to 300 μA $cm^{-2}$ for 150CISB, indicating its inability to cycled at so high current densities.

Given the η obtained for CISB electrolytes at 100 μA $cm^{-2}$ (from 117 to 250 mV for 125CISB and 150CISB, respectively), which is suitable for full-cell cycling, they were also tested in long term at that current density. As can be observed from Fig. 6, 125CISB exhibited a very stable cycling over 1500 h (η ~ 300 mV) in comparison with the trumpet shape of 100CISB and specially 150CISB in agreement with the ionic conductivity and impedance analysis of symmetric cells (see Table 2 below). 125CISB resulted in a total resistance of 2.77 kΩ while 100CISB (7.23 kΩ) and 150CISB (9.29 k) presented significantly higher resistances.

**Table 2.** Results of fitting the initial impedance of Li||Li cells.

| | $R_{bulk}$ (Ω) | $R_{int}$ (kΩ) | $R_{Li^+}$ (kΩ) |
|---|---|---|---|
| 1M LiFSI in IDE | 66 | 1.83 | - |
| 100CISB | 556 | 4.35 | 2.32 |
| 125CISB | 451 | 1.31 | 1.01 |
| 150CISB | 1550 | 3.89 | 3.84 |

Additionally, rectangular shape is still observed after 1500 h (see Fig. 6, inset) which is a good indicative of no degradation during cycling and homogeneous plating and stripping.

Example 5. Electrode preparation and full cell assembly

To probe the performance of 125CISB in real devices, LMFP were selected as cathodes due to the wide enough electrochemical stability of the studied system. LMFP cathode was prepared by dissolving first PVDF (5 wt. %) in NMP and subsequent addition of C65 (5 wt. %) and active material (90 wt. %). The honey-like mixture was homogenized with the aid of SpeedMixer at 3000 rpm before casting with an automatic doctor blade (Neurtek) at 40 mm $s^{-1}$ and 80 μm gap on carbon-coated aluminum foil. The loading of the resultant electrode was ~ 1 mAh $cm^{-2}$.

For the full cell assembly, the electrolyte membrane precursors were drop casted onto the cathode (8 mm ø) and they were irradiated with UV (365 nm) after 2 min of electrolyte diffusion through the electrode for 30 seconds. Afterwards, the cross-linked electrolyte|cathode membrane was faced to lithium metal anode (10 mm ø) in standard CR2032 cells.

The cobalt-free LMFP cathode was cycled at C/20 (44 μA $cm^{-2}$) at room temperature in the voltage range of 4.4-2.5 V vs $Li^+/Li^0$ showing a stable cycling, which is indeed improving over the first ten cycles (see Fig 7a-b). The initial coulombic efficiency of LMFP cell was 91.3 % and stabilized at 99.1 ± 0.6 % in the following cycles. Regarding the specific capacity, it reached 160 mAh $g^{-1}$ which is close to the theoretical capacity after stabilization. Additionally, the overpotential suffered in LMFP cell was also stabilized after the first ten cycles leading to 97 mV in the low-voltage plateau (centered at 3.50 V vs $Li^+/Li^0$) and 147 mV in the high-voltage plateau (centered at 4.05 V vs $Li^+/Li^0$). These results confirmed the suitability of 125CISB electrolyte to be employed in high-voltage devices.

## Claims

1. A mixture comprising:

   i) a monomer of formula

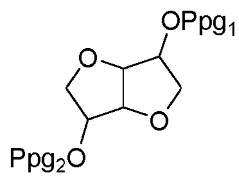

   wherein $Ppg_1$ and $Ppg_2$ are independently a polymerizable group, preferably $Ppg_1$ and $Ppg_2$ are independently selected from an olefin-containing or an isocyanate-containing polymerizable group; and
   ii) a lithium salt.

2. The mixture according to claim 1, wherein $Ppg_1$ and $Ppg_2$ are independently selected from acrylate or a C1-C6 alkyl substituted derivative thereof, styrene, diene, maleimide, norbornene, alkylene or isocyanate.

3. The mixture according to claim 1 or 2, wherein the monomer is selected from isosorbide dimethacrylate, isomannide dimethacrylate, and isoidide dimethacrylate; preferably the monomer is isosorbide dimethacrylate.

4. The mixture according to any one of claim 1 to 3, wherein the lithium salt is selected from $LiN(SO_2CF_3)_2$ (LiTFSI), $LiN(SO_2F)_2$ (LiFSI), $LiN(SO_2CF_3)(SO_2F)$, $LiN(C_2F_5SO_2)(SO_2F)$ $LiB(C_2O_4)_2$, $LiBF_4$, $LiBF_2(C_2O_4)$, $LiC(SO_2CF_3)_3$, $LiPF_3(C_2F_5)_3$, $LiCF_3SO_3$, n-BuLi, MeLi, t-BuLi, LiOMe or a mixture thereof.

**5.** The mixture according to any one of claims 1 to 4 further comprising a plasticizer.

**6.** The mixture according to claim 5, wherein the plasticizer is an ether, preferably selected from diethyl ether; dimethoxyethane (DME); tetraethylene glycol dimethyl ether (TEGDME); 1,4-dioxane; crown ethers; alkyl ether according to the formula:

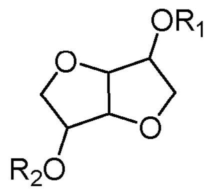

wherein $R_1$ and $R_2$ are independently selected from H, C1-C10 alkyl groups, fluorosulfate, sulfonamide, phenyl, provided that $R_1$ and $R_2$ cannot be both H; or a mixture thereof; preferably, wherein the alkyl ether according to the above formula is an alkyl ether of isosorbide, isomannide, or isoidide.

**7.** The mixture according to any of the preceding claims further comprising a co-monomer.

**8.** The mixture according to any of the preceding claims further comprising a polymerization initiator.

**9.** Method for preparing a solid or gel polymer electrolyte comprising polymerizing in the presence of a polymerization initiator, preferably by irradiating and/or heating, a monomer of formula:

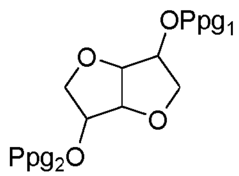

wherein $Ppg_1$ and $Ppg_2$ are independently a polymerizable group, preferably $Ppg_1$ and $Ppg_2$ are independently selected from an olefin-containing or isocyanate-containing polymerizable group, more preferably $Ppg_1$ and $Ppg_2$ are independently selected from acrylate or a C1-C6 alkyl substituted derivative thereof, styrene, diene, maleimide, norbornene, alkylene or isocyanate, wherein a lithium salt is added before, during or after polymerization of the monomer.

**10.** The method according to claim 9 comprising the steps of:

i) mixing the monomer, preferably selected from isosorbide dimethacrylate, isomannide dimethacrylate, and isoidide methacrylate, with the lithium salt and the polymerization initiator; and
ii) irradiating and/or heating the mixture from step i), causing thereby the polymerization of the monomer.

**11.** A solid or gel polymer electrolyte obtainable by the method according to claim 9 or 10.

**12.** A solid or gel polymer electrolyte comprising:

i) a polymer comprising monomeric units of formula:

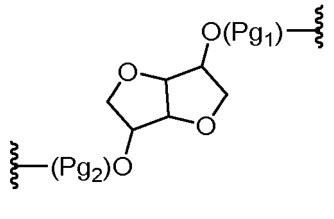

wherein $(Pg_1)$ and $(Pg_2)$ are independently obtained from the polymerization of a polymerizable group, preferably

from the polymerization of an olefin-containing or isocyanate-containing polymerizable group, more preferably from the polymerization of a polymeryzable group selected from acrylate or a C1-C6 alkyl substituted derivative thereof, styrene, diene, maleimide, norbornene, alkylene, or isocyanate; and
ii) a lithium salt.

**13.** The solid or gel polymer electrolyte according to claim 12, wherein the polymer further comprises a) monomeric units of a co-monomer other than the monomer and/or b) a plasticizer and/or c) a polymerization initiator.

**14.** An electrochemical cell or battery comprising a solid or gel polymer electrolyte according to any one of claims 11 to 13; preferably, comprising an anode, a solid or gel polymer electrolyte according to any one of claims 11 to 13, a cathode and, optionally, a separator; preferably wherein the electrochemical cell or battery is a lithium ion cell or battery.

**15.** Use of the electrochemical cell or battery according to claim 14 for storing energy, preferably in a vehicle, an electronic device or an electrical grid.

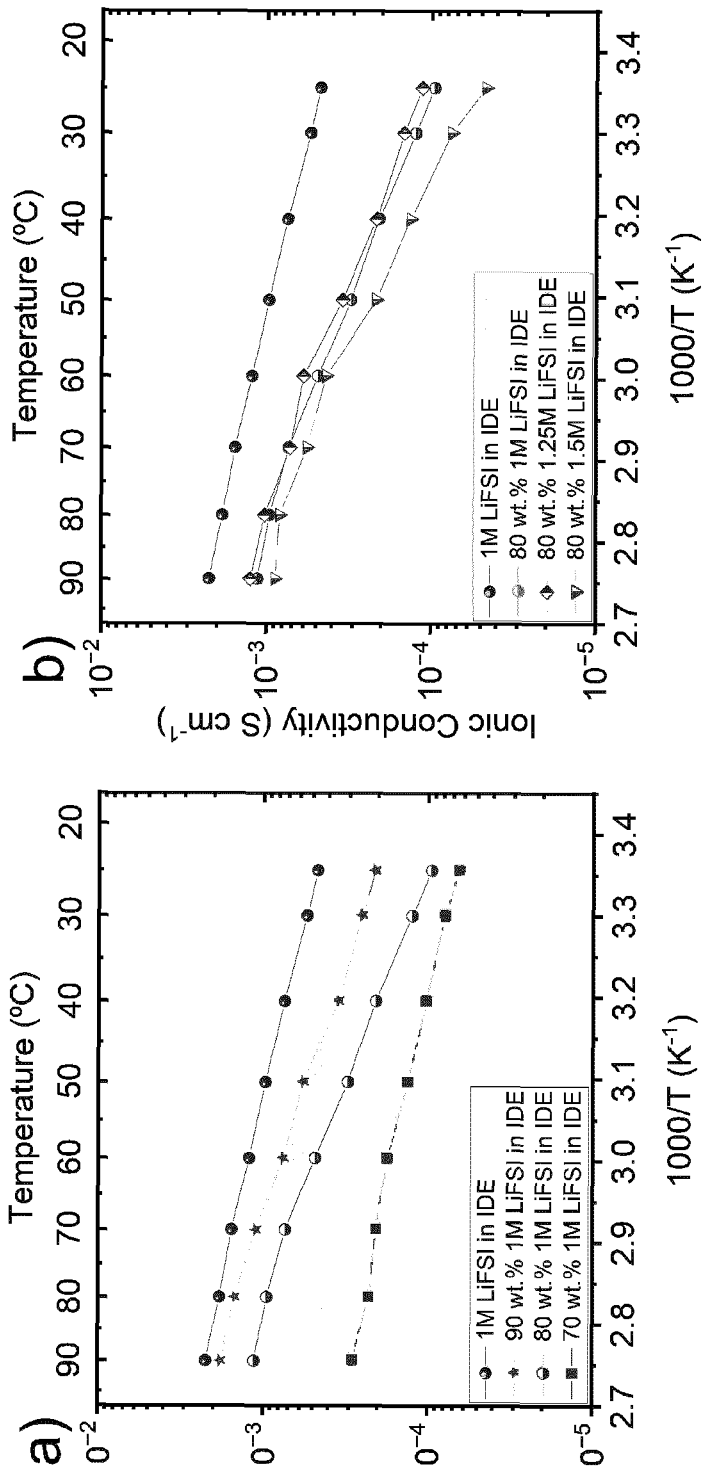
a)
b)
Temperature (ºC)
1000/T (K$^{-1}$)
Ionic Conductivity (S cm$^{-1}$)
1M LiFSI in IDE
90 wt.% 1M LiFSI in IDE
80 wt.% 1M LiFSI in IDE
70 wt.% 1M LiFSI in IDE
80 wt.% 1.25M LiFSI in IDE
80 wt.% 1.5M LiFSI in IDE

Figure 1

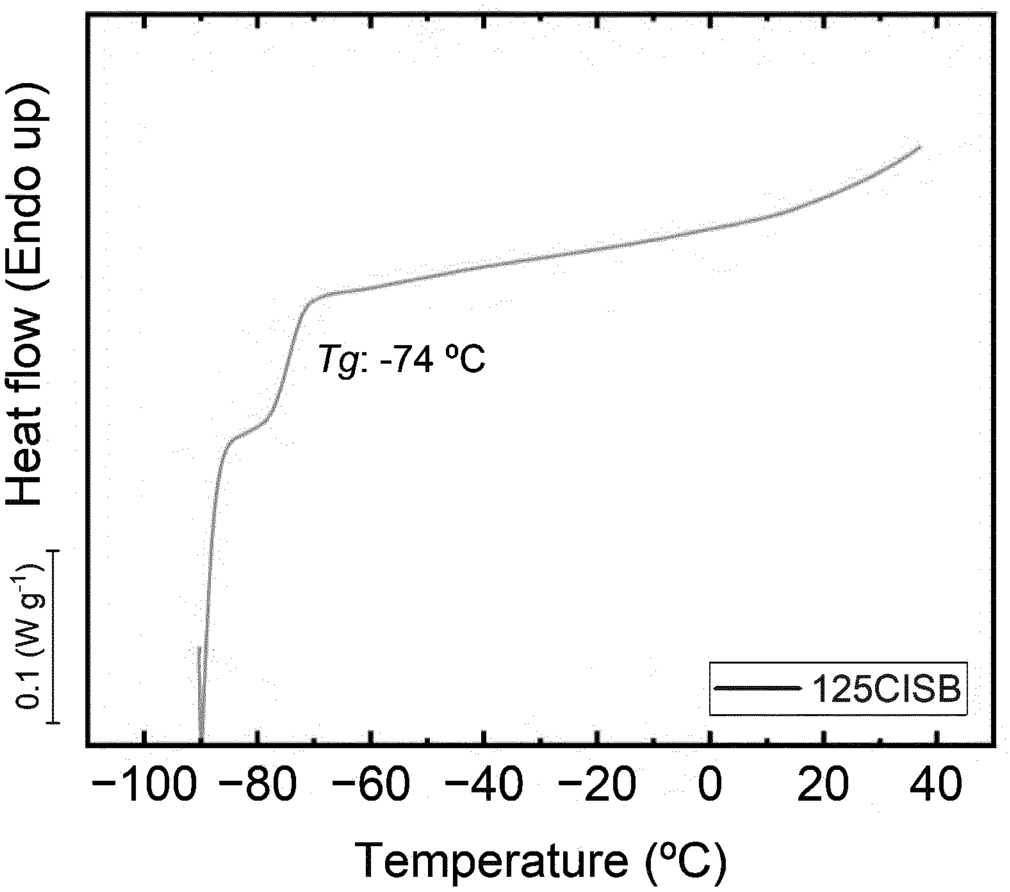
Heat flow (Endo up)
0.1 (W g-1)
Tg: -74 ºC
125CISB
−100 −80 −60 −40 −20 0 20 40
Temperature (ºC)

Fig. 2

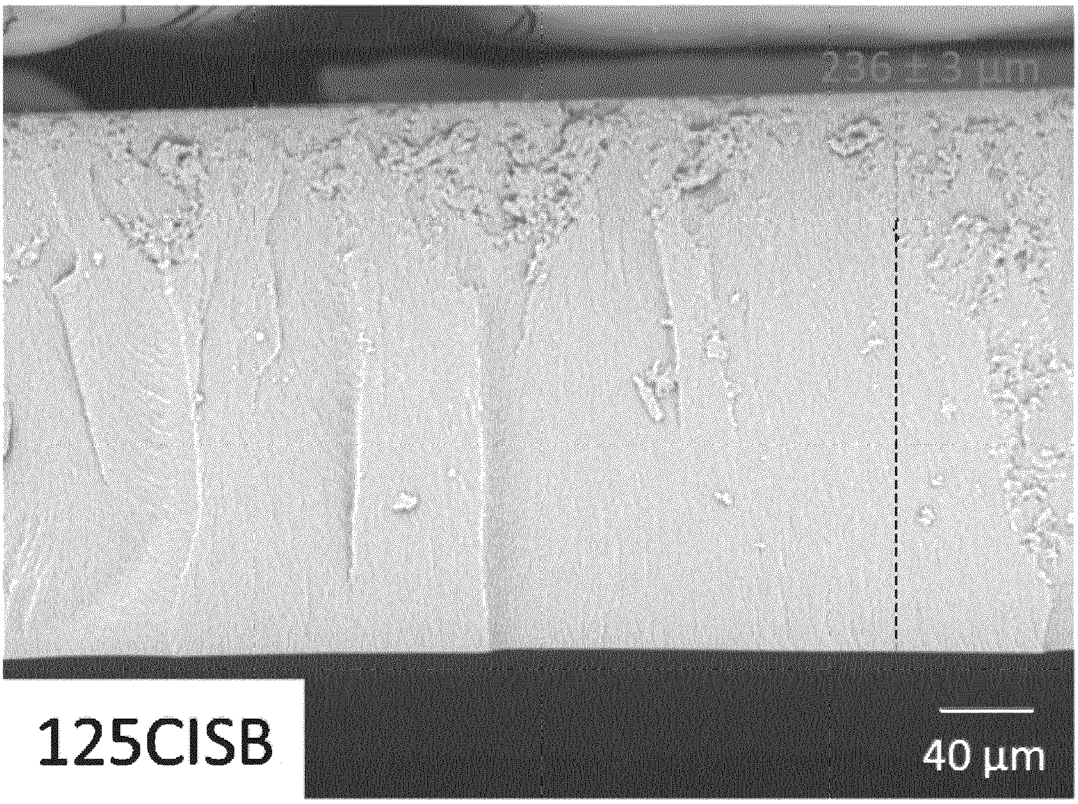


Figure 3

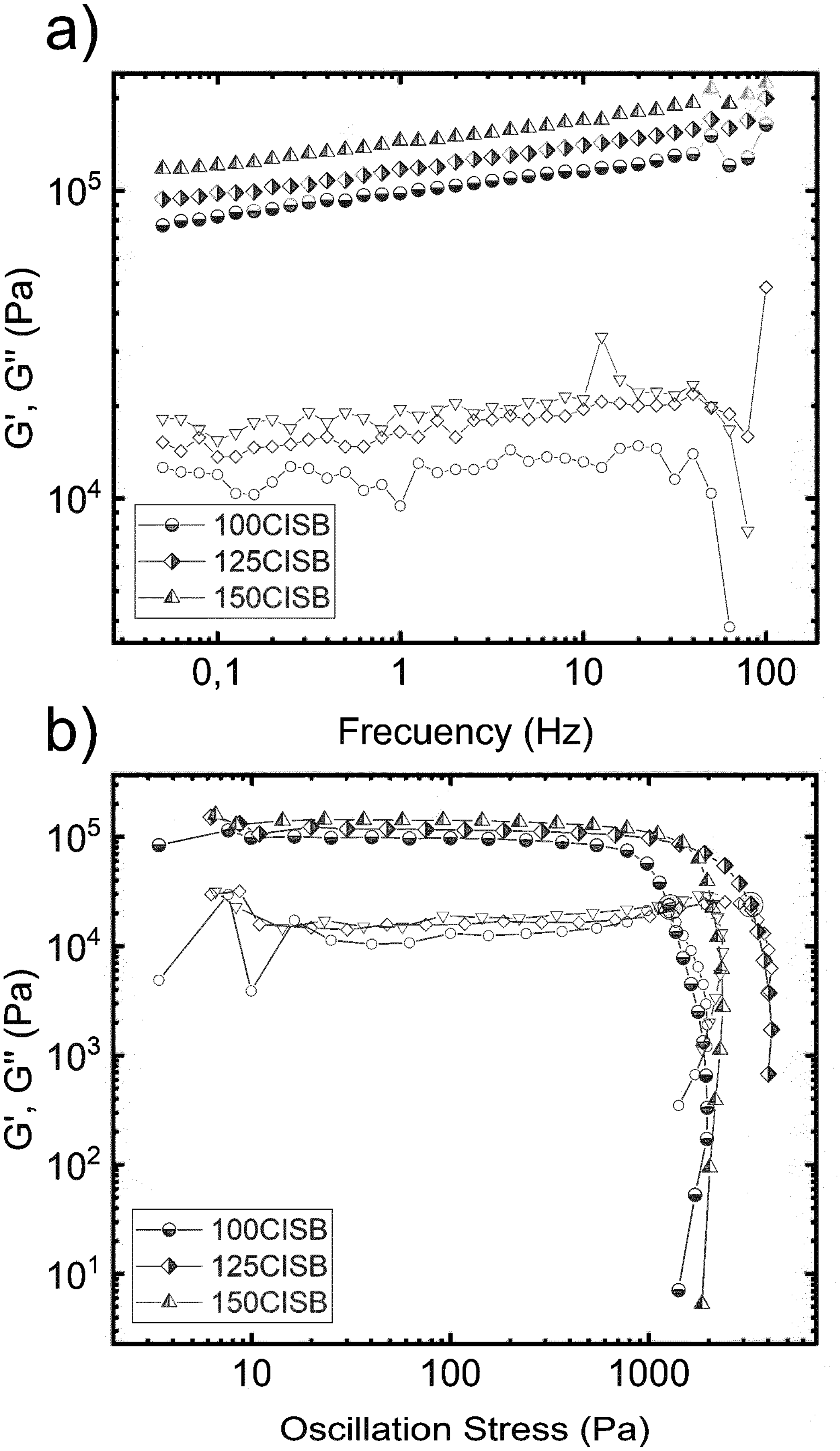
a)
G', G'' (Pa)
10^5
10^4
0,1
1
10
100
Frecuency (Hz)
100CISB
125CISB
150CISB
b)
G', G'' (Pa)
10^5
10^4
10^3
10^2
10^1
10
100
1000
Oscillation Stress (Pa)
100CISB
125CISB
150CISB

**Figure 4**

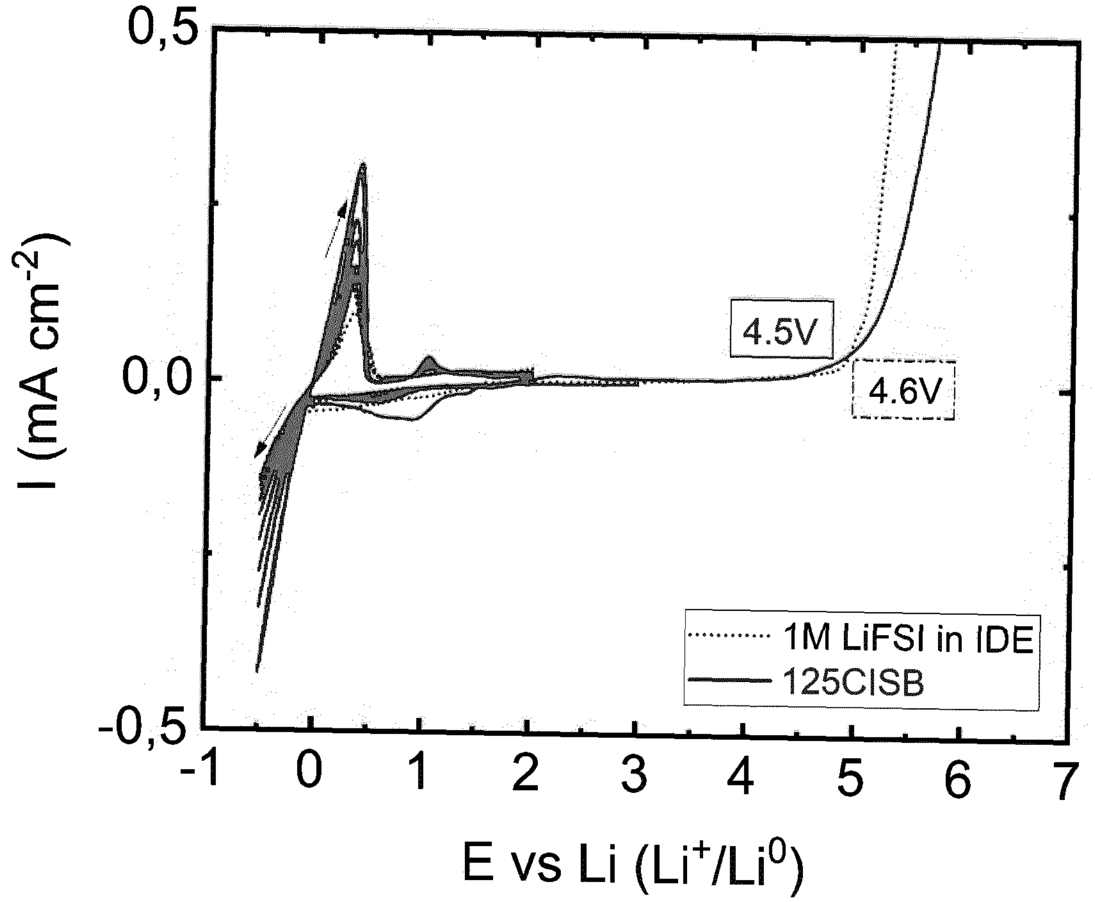
0,5
0,0
-0,5
I (mA cm-2)
-1
0
1
2
3
4
5
6
7
E vs Li (Li+/Li0)
4.5V
4.6V
1M LiFSI in IDE
125CISB

**Figure 5**

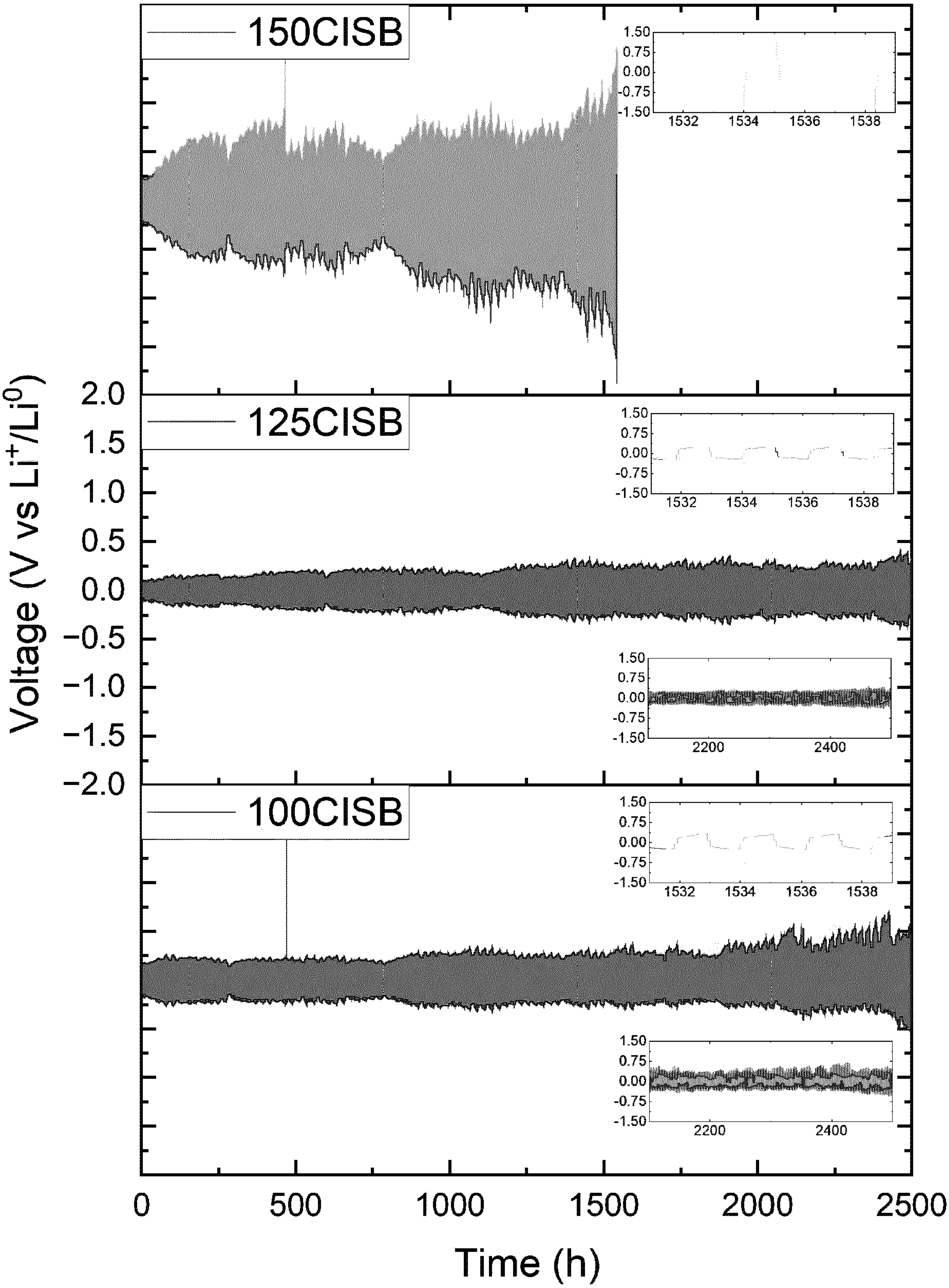
150CISB
125CISB
100CISB
Voltage (V vs Li+/Li0)
2.0
1.5
1.0
0.5
0.0
−0.5
−1.0
−1.5
−2.0
1.50
0.75
0.00
-0.75
-1.50
1532
1534
1536
1538
2200
2400
0
500
1000
1500
2000
2500
Time (h)

Figure 6

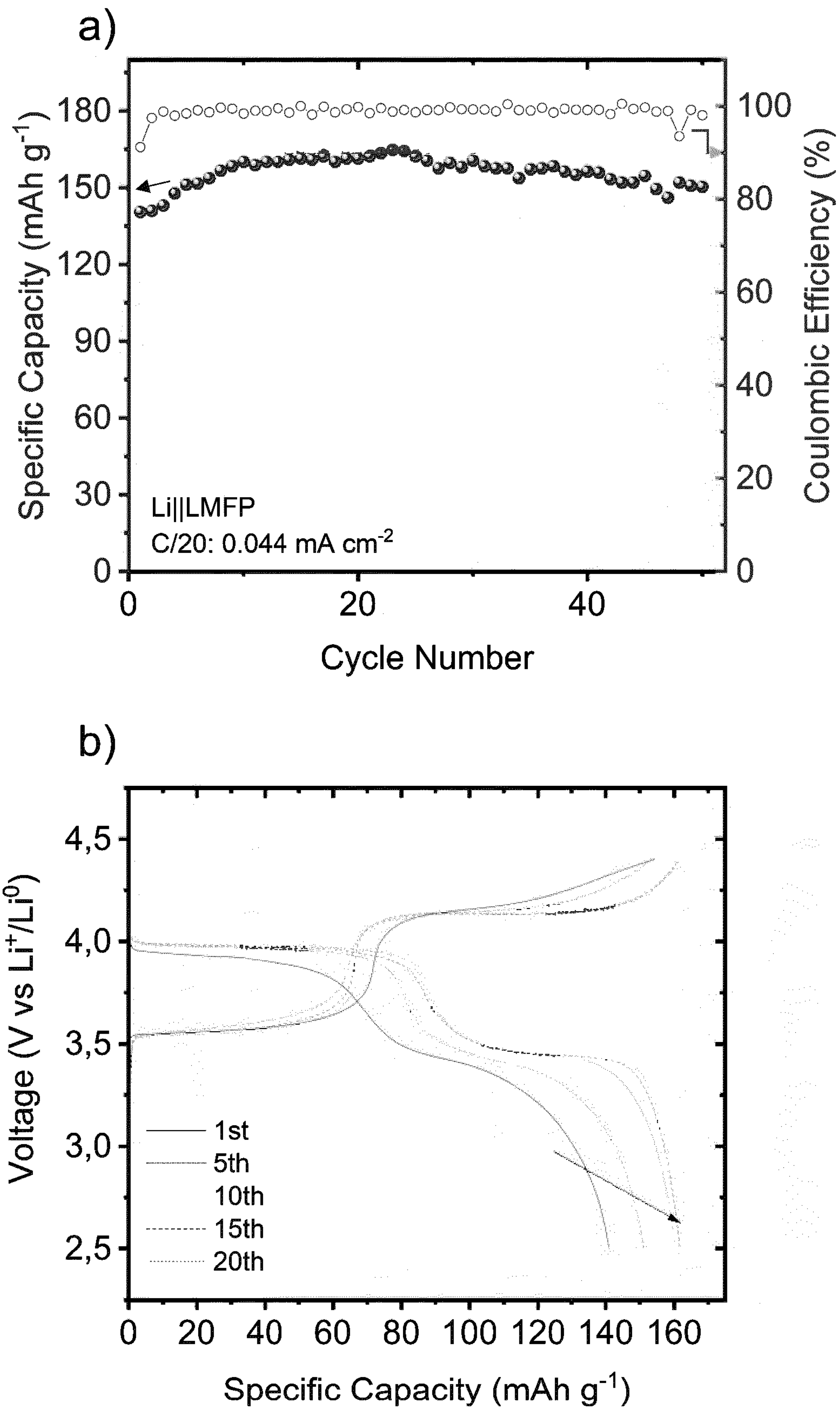
a)
Specific Capacity (mAh g-1)
Coulombic Efficiency (%)
Cycle Number
Li||LMFP
C/20: 0.044 mA cm-2
180
150
120
90
60
30
0
100
80
60
40
20
0
0
20
40
b)
Voltage (V vs Li+/Li0)
Specific Capacity (mAh g-1)
4,5
4,0
3,5
3,0
2,5
0
20
40
60
80
100
120
140
160
1st
5th
10th
15th
20th

Figure 7

Europäisches Patentamt
European Patent Office
Office européen des brevets

# EUROPEAN SEARCH REPORT

Application Number
EP 25 38 2059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/041169 A1 (FUERTES PATRICK [FR] ET AL) 16 February 2012 (2012-02-16) | 1,8-13 | INV. H01M10/0525 C07D493/04 H01M10/0565 C08F122/10 C08L33/06 |
| A | * abstract * * example 9 * ----- | 2-7 | |
| X | CN 107 394 264 A (UNIV ANHUI) 24 November 2017 (2017-11-24) * abstract * * paragraph [0004] * * example 1 * ----- | 9,11-15 | |
| A | WO 2011/048739 A1 (DAI ICHI KOGYO SEIYAKU CO LTD [JP]; TAKAMURA NAOHIRO [JP] ET AL.) 28 April 2011 (2011-04-28) * abstract * * example 1 * ----- | 1-15 | |

TECHNICAL FIELDS SEARCHED (IPC)

H01M
C07D
C08F
C08L

The present search report has been drawn up for all claims

1

| Place of search | Date of completion of the search | Examiner |
|---|---|---|
| The Hague | 3 July 2025 | Rosciano, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 25 38 2059

This annex lists the patent family members relating to the patent documents cited in the above-mentioned European search report. The members are as contained in the European Patent Office EDP file on
The European Patent Office is in no way liable for these particulars which are merely given for the purpose of information.

03-07-2025

| Patent document cited in search report | Publication date | Patent family member(s) | Publication date |
|---|---|---|---|
| US 2012041169 A1 | 16-02-2012 | AU 2010302454 A1 | 13-10-2011 |
| | | CA 2756160 A1 | 07-04-2011 |
| | | CN 102365286 A | 29-02-2012 |
| | | EP 2483276 A1 | 08-08-2012 |
| | | ES 2613061 T3 | 22-05-2017 |
| | | FR 2950892 A1 | 08-04-2011 |
| | | JP 5801813 B2 | 28-10-2015 |
| | | JP 2013506645 A | 28-02-2013 |
| | | KR 20120093155 A | 22-08-2012 |
| | | US 2012041169 A1 | 16-02-2012 |
| | | WO 2011039483 A1 | 07-04-2011 |
| CN 107394264 A | 24-11-2017 | NONE | |
| WO 2011048739 A1 | 28-04-2011 | CN 102548997 A | 04-07-2012 |
| | | JP 5270510 B2 | 21-08-2013 |
| | | JP 2011084535 A | 28-04-2011 |
| | | KR 20120063490 A | 15-06-2012 |
| | | TW 201118066 A | 01-06-2011 |
| | | WO 2011048739 A1 | 28-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHAI, P. et al.** *Adv. Energy Mater.*, 2020, vol. 10 (34), 1-32
- **ZHANG, C. et al.** *J. Mater. Chem. A*, 2021, vol. 9 (23), 13388-13401
- **LI, S. et al.** *Adv. Mater.*, 2018, vol. 30 (17), 1-29
- **ADENUSI, H. et al.** *Adv. Energy Mater.*, 2023, vol. 13 (10)
- **ASLAM, M. K. et al.** *Nano Energy*, 2021, vol. 86, 106142
- **LI, S. et al.** *Energy Storage Mater.*, 2020, vol. 32, 306-319
- **YANG, H. et al.** *Energy Storage Mater.*, 2020, vol. 30, 113-129
- **JOHNSON, N. M. et al.** *ACS Energy Lett.*, 2022, vol. 7 (2), 897-905
- **KANG, S. et al.** *Energy Mater.*, 2023, vol. 3 (5)
- From Molecular Systems to Molecular Devices. **HONDA**. Functionality of Molecular Systems. Springer-Verlag, 1999, vol. 2
- **HATADA et al.** Macromolecular design of polymeric materials. Marcel Dekker, Inc., 1997
- **LI et al.** *Chem Soc Rev*, 2017, vol. 46, 3006-3059
- **LYU et al.** *Sustainable Materials and Technologies*, 2019, vol. 21, e00098
- *CHEMICAL ABSTRACTS*, 1333-86-4
- **CHAKRABORTY, S. et al.** *ACS Appl. Energy Mater.*, 2022, vol. 5 (1), 852-861
- **DEL OLMO, R. et al.** *J. Power Sources*, 2024, vol. 621, 235233
- **BRUCE, P. G. et al.** *Solid State Ionics*, 1988, vol. 28-30, 918-922